# EUROPEAN PATENT SPECIFICATION

(11) **EP 1 922 087 B1**
(45) Date of publication and mention of the grant of the patent: **17.08.2011**
(21) Application number: 06775676.7
(22) Date of filing: 05.09.2006
(51) Int. Cl.: A61K 47/48, A61P 35/00, C08F 220/58, C08F 8/34

(54) **METHOD FOR THE PREPARATION OF POLYMERIC CONJUGATES OF DOXORUBICIN WITH PH-CONTROLLED RELEASE OF THE DRUG**
VERFAHREN ZUR HERSTELLUNG VON POLYMER-KONJUGATEN VON DOXORUBICIN MIT PH-KONTROLLIERTER FREISETZUNG DES ARZNEIMITTELS
PROCEDE POUR LA PREPARATION DE CONJUGUES POLYMERES DE DOXORUBICINE A LIBERATION DE MEDICAMENT A PH REGULE

(30) Priority: 05.09.2005 CZ 20050558
(43) Date of publication of application: 21.05.2008
(73) Proprietor: Zentiva, k.s., Dolni Mecholupy 102 37 Praha 10 (CZ)
(72) Inventor: ETRYCH, Tomas, 252 10 Klinec 213 (CZ); CHYTIL, Petr, 160 00 Praha 6 (CZ); STUDENOVSKY, Martin, 169 00 Praha 6 (CZ); PECHAR, Michal, 120 00 Praha 2 (CZ); ULBRICH, Karel, 140 00 Praha 4 (CZ); RIHOVA, Blanka, 140 00 Praha 4 (CZ)
(74) Representative: Jirotkova, Ivana
(86) International application number: PCT/CZ2006/000056
(87) International publication number: WO 2007/028347

(56) References cited:
- WO-A-03/053473
- WO-A-2004/045647
- WO-A-2005/007798
- WO-A-2005/117932
- WO-A-2005/123676
- WO-A-2006/003014
- ETRYCH, T. ET AL: "Synthesis of HPMA copolymers containing doxorubicin bound via a hydrazone linkage. Effect of spacer on drug release and in vitro cytotoxicity" MACROMOL. BIOSCI., vol. 2, 2002, pages 43-52, XP008076237
- BOSE A K ET AL: "Microwave enhanced Akabori reaction for peptide analysis" JOURNAL OF THE AMERICAN SOCIETY FOR MASS SPECTROMETRY, ELSEVIER SCIENCE INC., NEW YORK, NY, US, vol. 13, no. 7, July 2002 (2002-07), pages 839-850, XP004371091 ISSN: 1044-0305
- MRKVAN ET AL: "Chemotherapy based on HPMA copolymer conjugates with pH-controlled release of doxorubicin triggers anti-tumor immunity" JOURNAL OF CONTROLLED RELEASE, ELSEVIER, AMSTERDAM, NL, vol. 110, no. 1, 10 December 2005 (2005-12-10), pages 119-129, XP005192557 ISSN: 0168-3659

## Description

### Technical Field

The invention concerns a method of preparation of water-soluble polymeric anticancer drugs according to claim 1 enabling targeted transport and controlled release of cytostatics in the organism, preferentially in tumour tissue and tumour cells. The polymeric conjugates are for use in the targeted therapy of tumour diseases in human medicine.

### Background Art

The development of new pharmacologically active substances, particularly anticancer drugs, has increasingly been focused on such forms that-enable specific action of the active substance only in a specific tissue, or even only in a specific cell type. Natural or synthetic macromolecules - polymers - have increasingly been used for the preparation of such substances. Many anticancer polymeric conjugates have been prepared and studied, and it has been shown that in most cases it is necessary that the cytotoxic substance be released from its polymeric form if the polymeric form of the drug is to be pharmacologically effective. It has further been shown that a suitable molecular weight of the polymeric carrier can ensure preferential deposition of polymeric drug in the tumour tissue of many solid tumours (the socalled EPR effect) [Maeda et al. 2000]. The release of the cytostatic agent from its polymeric carrier can be ensured using a biodegradable link, used for binding the drug to the polymer, the degradation of which in the target tissue leads to targeted and controlled activation of the drug preferentially in said tissue. Polymeric drugs based on N-(2-hydroxypropyl)-methacrylamide (HPMA) copolymers are an important group of such drugs. A very good overview of the research results in this area up to the present can be found in G. S. Kwon's monograph and in the publication of J. Kope 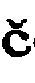 ek et al. [Kope 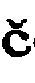 ek et al. 2000, Kwon 2005]. Recently, studies on the action of polymeric drugs have been published where the anticancer drug doxorubicin is bound to a polymeric carrier, based on HPMA copolymers, by means of a hydrolytically unstable hydrazone bond [Etrych et al. 2001 and 2002, Ríhová et al. 2001, Ulbrich et al. 2003, 2004], and these substances have been patented [Ulbrich et al.]. These drugs showed a significant decrease in side, especially toxic, effects on the healthy organism, while significantly increasing the anticancer effect when compared with commonly used cytostatics [Ríhová et al. 2001, Kovár et al. 2004, Hovorka et al. 2002].

The synthesis of such conjugates was carried out first by polymer-analogical reaction of polymeric 4-nitrophenyl esters (ONp) with hydrazine, and later by copolymerization of HPMA with *N*-Boc (t-butyloxycarbonyl) protected methacryloylated hydrazides. Neither of the methods led to the formation of well-defined preparations (in the case of ONp esters, transfer reactions and hydrolysis of part of ONp groups during hydrazinolysis take place; in the case of Boc-hydrazides, degradation reactions occur during the deprotection of hydrazide groups), and neither of the methods enabled a choice of a wide range of molecular weights of the polymeric chain; the synthesis did not make it possible to prepare large batches and the reproducibility of the preparation of individual batches was relatively low. Moreover, the syntheses included several steps, which increased their time and financial demands.

### Disclosure of Invention

The present invention provides an optimized and reproducible method of preparation of polymeric cytostatics based on HPMA copolymers containing doxorubicin bound by a pHlabile hydrazone bond to the polymeric carrier, which eliminates practically all the aforementioned shortcomings of earlier published preparation methods; in particular, it makes it possible to increase yields during the synthesis of both monomers and the polymeric precursor, to control precisely the molecular weights of the polymeric precursors and of the final product, the structure is, due to advantageous copolymerization parameters, well defined, the synthesis is significantly easier and cheaper, a scale-up to large batches is possible, and the reproducibility of the synthesis is very good. The antitumour activity of the polymeric cytostatics prepared according to the invention is the same as, or even better than, that of cytostatics prepared by prior methods.

The subject matter of the invention consists in a method of preparation of a polymeric conjugate of HPMA copolymer containing doxorubicin bound to the polymer by means of various links containing hydrolytically cleavable hydrazone bonds. The method of the preparation is based on a three-step synthesis comprising the synthesis of monomers, the synthesis of polymeric precursors, and the final binding of doxorubicin to the polymeric carrier by a covalent hydrazone bond.

***The synthesis of monomers*** starts with the synthesis of HPMA monomer according to the previously described method [Ulbrich 2000]. The synthesis of methacryloyl-(aminoacyl)hydrazines differing in the structure of the acyl component was very similar for all the monomers prepared and starts with methacryloylation of the methyl ester hydrochloride of the respective amino acid or oligopeptide with methacryloyl chloride, carried out in dichloromethane in the presence of anhydrous sodium carbonate. The resulting product was converted into the methacryloylated aminoacylhydrazine by hydrazinolysis of the respective methyl ester with hydrazine hydrate, carried out in methanolic solution in the presence of NaOH. Glycyl, glycylglycyl, β-alanyl, 6-aminohexanoyl, 4-aminobenzoyl, or a complex acyl issuing from oligopeptides GlyPheGly, GlyLeuGly, or GlyPheLeuGly, was advantageously used as the aminoacyl in the methacryloyl(aminoacyl)hydrazines. As examples of the synthesis of a methacryloyl(aminoacyl)hydrazine, Example 1 shows the synthesis of 6methacroyl(aminohexanoyl)hydrazine as an example of the synthesis of a simple acyl (spacer), of methacroylglycylglycylhydrazine as a monomer with a dipeptide spacer, and of methacroylglycylphenylalanylleucylglycylhydrazine as a monomer with an enzymatically degradable oligopeptide.

***The synthesis of polymeric precursors** -* HPMA copolymers with methacryloylated aminoacylhydrazines - is based on direct radical copolymerization of HPMA with the corresponding methacryloylated hydrazines. The polymerization is carried out in a solution using methanol, ethanol, dimethylsulfoxide, or dimethylformamide as the polymerization medium. In both cases, the polymerization is initiated by thermally decomposable initiators of radical polymerization based on azo or peroxy initiators. Preferably, azobis(isobutyronitrile) (AIBN), azobis(isocyanovaleric acid) (ABIC), or diisopropyl percarbonate (DIP) were used. The temperature of the polymerization depends on the initiator and solvent used (AIBN, ABIC in methanol, ethanol, DMF, and DMSO, 50 to 60 °C; DIP, 40 to 50 °C). The polymerization usually takes 15 to 18 hours. The preparation of all the polymeric precursors by radical polymerization is analogical; examples of copolymerization of HPMA with methacrylated hydrazides are given in Examples 2a to 2c. When compared with the earlier used hydrazinolysis of reactive esters or copolymerization of Boc-protected hydrazides, the direct copolymerization leads to well-defined and reproducibly prepared polymers that can be prepared in large batches and in high yields.

***The binding of doxorubicin to the polymeric precursor*** issues from the binding reaction of doxorubicin hydrochloride to the polymeric acylhydrazine resulting in a hydrazone bond. The reaction is advantageously carried out in methanol, catalyzed by a defined amount of acetic acid. The reaction can also be carried out in dimethylsulfoxide, dimethylformamide, dried ethanol, and dimethylacetamide. When using solvents other than methanol, the reaction proceeds well, but the yields are lower. The influence of the structure of the link on the course of the binding reaction is minimal. To achieve the optimum yield of the binding reaction and the minimum amount of unbound doxorubicin in the product, it is important, in all cases, to maintain the following concentrations of the polymer and acetic acid in the reaction mixture: concentration of the polymer 170 mg/ml, concentration of acetic acid 55 mg/ml. The optimum reaction time is 22 hours at 25 °C. The polymeric drug is isolated from the reaction mixture by precipitation into ethyl acetate and reprecipitation from methanol again into ethyl acetate. The preparation of polymeric doxorubicin bound to a polymeric precursor - copolymer poly(HPMA-co-MA-AH-NHNH₂) - by a hydrazone bond (PHPMA-AH NH-N=DOX) is given in Example 3.

The preparation also includes, although it is not necessary, ***final purification of the conjugate*** from free unbound drug by gel filtration using a Sephadex LH-20 column with methanol as the mobile phase.

### Brief Description of Drawings

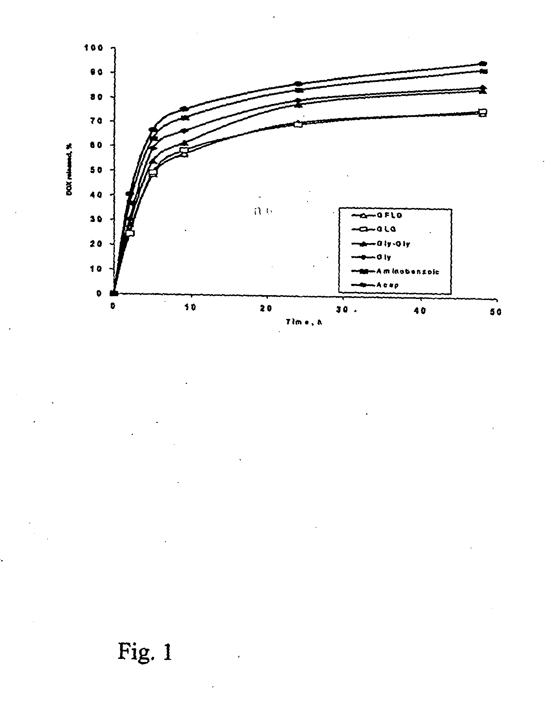

Fig. 1 presents a graph showing the release of DOX from polymeric conjugates differing in the structure of the link used between the drug and the polymer. Temperature: 37 °C, phosphate buffer, pH 5.5. GFLG is a link formed by the sequence -GlyPheLeuGly-, GLG is -GlyLeuGly-Aminobenzoic is 4-aminobenzoyl, and Acap is 6-aminohexanoyl.

### Examples

### Example 1: Synthesis of monomers

HPMA was prepared according to the previously described method [Ulbrich et al. 2000]. Elementary analysis: Calculated: C 58.8 %, H 9.16 %, N 9.79 %. Found: C 58.98 %, H 9.18 %, N 9.82 %. The product was chromatographically pure.

6-(Methacroylamino)hexanoylhydrazine (MA-AH-NHNH₂) Methyl(6-aminohexanoate)hydrochloride (30 g, 0.165 mol) was, under vigorous stirring at room temperature, dissolved in 350 ml of dichloromethane with the addition of ca. 100 mg of hydroquinone. The solution was cooled to 10 to 15 °C, anhydrous sodium carbonate (50 g, 0.48 mol) was added, the temperature was reduced to 5 to 10 °C, and then a solution of methacroyl chloride (17.3 g, 0.165 mol (eq.)) in 100 ml of dichloromethane was added dropwise at such a rate that the temperature of the reaction mixture does not exceed 15 °C. After consumption of all methacroyl chloride, the mixture was stirred at 15 to 20 °C for further 45 minutes, then the cooling bath was removed, the suspension was stirred for another 20 minutes, sucked off in sintered glass filter No. 3, washed with 300 ml of dichloromethane, and the filtrate was evaporated to dryness in a rotary vacuum evaporator. The evaporation residue was dissolved in 150 ml of methanol, then hydrazine hydrate (13 ml ≈ 13.4 g, 0.267 mol) and NaOH (1.5 g, 37.5 mmol) were added, and the reaction mixture was stirred at room temperature for 3 hours. After the hydrazinolysis was completed, pH of the solution was adjusted to 6.2 to 6.5 by adding 35% HCl (ca. 12 ml), 300 g of anhydrous sodium sulfate was added, and the mixture, including the desiccant, was evaporated to dryness. 500 ml of dichloromethane was added to the evaporation residue, the suspension was stirred vigorously for 2 hours, sucked off in sintered glass filter No. 4, thoroughly washed with another 500 ml of dichloromethane, and the filtrate was concentrated to 150 to 200 ml in an evaporator. The solution was diluted with 1500 ml of ethyl acetate, concentrated for crystallization to the volume of 300 to 350 ml in an evaporator, and allowed to crystallize in a freezing box for 24 hours. The product was sucked off, washed with a small amount of cold ethyl acetate, and dried in vacuo. The yield after the first crystallization was 29.5 g of the product (84 %) with a melting point of 79 to 81 °C; repeated crystallization using the same method gave 27.0 g of the product (77 %) with a melting point of 80 to 82 °C. Elementary analysis: Calculated: C 56.32 %, H 8.98 %, N 19.70 %. Found: C 56.49 %, H 8.63 %, N 19.83 %. H-NMR 300 MHz (CDCl₃, 297 K): 1.35 m (2H, CH₂(CH₂)₂-N); 1.50-1.69 m (4H, CH₂CH₂CH₂CH₂-N); 1.95 dd (3H, CH₃); 2.17 t (2H, ((C=O)-CH₂); 3.26 dt (2H, N-CH₂); 3.91 s (2H, NH₂); 5.30 t (1H, C=CH₂ E); 5.67 t (1H, C=CH₂ Z); 6.10 s (1H, NHNH₂); 7.45 s (1H, NH-CH₂). The product was chromatographically pure, 1 peak at 4.72 min (reverse phase column Tessek SGX C₁₈ (7 µm, 125 x 4 mm), flow rate 0.5 ml/min, gradient from 40 to 100 % of solution B in 35 min (solution A: 10 % methanol, 89.9 % water, 0.1 % trifluoroacetic acid (TFA); solution B: 89.9 % methanol; 10 % water; 0,1 % TFA), UV detection).

Methacroylglycylglycylhydrazine (MA-GlyGly-NHNH₂) The preparation of MA-GlyGly-NHNH₂ was carried out under similar conditions as that of MA-AH-NHNH₂. Glycylglycine methyl ester (21.4 g, 0.1 mol) was dissolved in 250 ml of dichloromethane with the addition of 60 mg of hydroquinone. After cooling to ca. 12 °C, anhydrous sodium carbonate (30.2 g, 0.29 mol) was added, and, under cooling to 5 to 10 °C, a solution of methacroyl chloride (10.4 g, 0.1 mol) in 60 ml of dichloromethane was slowly added dropwise. After completion of the reaction and filtering and washing of the precipitate with ca. 230 ml of dichloromethane, the solvent was completely evaporated. The evaporation residue was dissolved in 120 ml of methanol, and hydrazinolysis with hydrazine hydrate (7.9 ml ≈ 8.1 g, 0.166 mol) was carried out in the presence of NaOH (0.91 g, 22.7 mmol). After the hydrazinolysis was completed, pH of the solution was adjusted to 6.2 to 6.5 by adding 35% HCl (ca. 12 ml), 230 g of anhydrous sodium sulfate was added, and the mixture, including the desiccant, was evaporated to dryness. After extraction with 300 ml of dichloromethane, the suspension was sucked off in a sintered glass filter, washed with another 300 ml of dichloromethane, and the filtrate was concentrated to ca. 120 ml. The solution was diluted with 900 ml of ethyl acetate and, after concentration to 250 ml in an evaporator, allowed to crystallize in a freezing box. After recrystallization; the product was isolated by filtration, washed with a small amount of cold ethyl acetate, and dried in vacuo. The yield of the product was 15.0 g (70 %), m.p. 172-174 °C. Elementary analysis: Calculated: C 44.86 %, H 6.54 %, N 26.16 %. Found: C 45.01 %, H 6.57 %, N 26.02 %. The product was chromatographically pure, one peak at 21.97 min.

Methacroylglycylphenylalanylleucylglycylhydrazine (MA-Gly-D,L-PheLeuGly-NHNH₂) The synthesis of this monomer was carried out in an analogical way as in both the previous cases (see above). The composition of the reaction mixture and the conditions were as follows: Glycylphenylalanylleucylglycine methyl ester (22.32 g, 0.05 mol), dichloromethane 510 ml (270 + 240 ml), hydroquinone (60 mg), sodium carbonate (15.1 g, 0.145 mol), and methacroyl chloride 5.2 g (0.05 mol) were used for the preparation of methacrylated methyl ester. 4.1 g (0.085 mol) of hydrazine hydrate in 120 ml of methanol and 0.46 g (11.3 mmol) of NaOH was used for the hydrazinolysis. 210 g of anhydrous sodium sulfate was used for the drying, and 2 x 290 ml of dichloromethane was used for the extractions. The product was crystallized from a mixture dichloromethane - ethyl acetate. The yield of the product was 60 %, m.p. 139 to 140 °C. Elementary analysis: Calculated: C 58.10%, H 7.36 %, N 17.68 %. Found: C 58.21 %, H 7.39 %, N 17.54 %. The product was chromatographically pure, two peaks having the same area at 19.39 (L-Phe containing monomer) and 19.91 min (D-Phe).

### Example 2a: Synthesis of a polymeric precursor - copolymer of HPMA with 6(methacroylamino)hexanoylhydrazine (poly(HPMA-co-MA-AH-NHNH₂))

Copolymer poly(HPMA-co-MA-AH-NHNH₂) was prepared by radical solution copolymerization of HPMA and MA-AH-NHNH₂ initiated by AIBN in methanol at 60 °C. 122.8 g of HPMA and 13.94 g of MA-AH-NHNH₂ (18 wt % of monomers) were dissolved in 780 ml of methanol, and 6.06 g of AIBN (0.8 wt %) was added to the solution. After filtration, the polymerization mixture was placed, in an argon atmosphere, into a polymerization reactor (volume 1.51), situated in a thermostat. The polymerization mixture was stirred at a higher rotation (about 100 rpm). Nitrogen was introduced over the surface still for several minutes. The temperature of the polymerization mixture was set to 60 °C, and the polymerization proceeded under stirring (50 rpm) in a nitrogen atmosphere. The nitrogen was drawn off through a bubbling device.

After 17 hours, the polymerization mixture was taken out of the thermostat, cooled in a bath to room temperature, and the polymer was isolated by precipitation into ethyl acetate (81 altogether). The precipitated polymer was allowed to sediment for ca. 0.5 hours, the solution over the precipitate was removed by suction, and the polymer was isolated by filtration in sintered glass filter S4. The precipitate was washed with ethyl acetate, transferred into large Petri dishes, and dried at room temperature in vacuo using a membrane vacuum pump for ca. 1 hour.

The polymer was, using ultrasound, dissolved in 550 ml of methanol (one-litre Erlenmeyer flask) and precipitated into 7.51 of ethyl acetate in the same way as during the first isolation. The precipitated polymer was, after being allowed to sediment for ca. 0.5 hours, isolated by filtration in sintered glass filter S4, washed with ethyl acetate, and dried until constant weight using a membrane vacuum pump (ca. 5 hours), and the drying process was completed using an oil diffusion pump.

Characterization of the copolymer:
Yield 114 g (83 %), the content of hydrazide groups 5.83 mol %, molecular weight M_{w} = 28500 g/mol, polydispersity index Iₙ =1.9.

### Example 2b: Synthesis of a polymeric precursor - copolymer of HPMA with methacroylglycylglycylhydrazine (poly(HPMA-co-MA-GlyGly-NHNH₂))

The configuration and the polymerization procedure were the same as in Example 2a, the difference being in the composition of the polymerization mixture. The composition of the polymerization mixture was as follows: HPMA 10 g (70 mmol), MA-GlyGly-NHNH₂ 1.5 g (7 mmol), diisopropyl percarbonate 1.15 g (0.91 wt %), and dimethylformamide 115 ml. The temperature of the polymerization was 50 °C, and the polymerization took 16 hours. The polymerization solution was, before precipitation into an excess of ethyl acetate, concentrated to ca. 2/3 of its original volume in a vacuum evaporator, and the precipitation of the polymeric product was carried out into a 20-fold volume of the precipitant. The polymer was depleted from low-molecular admixtures by precipitation from methanol into ethyl acetate. The yield was 8.5 g (70 %), the content of hydrazide groups 9.5 mol %, molecular weight M_{w} = 41700 g/mol, polydispersity index In = 2.1.

### Example 2c: Synthesis of a polymeric precursor - copolymer of HPMA with methacroylglycylphenylalanylleucylglycylhydrazine (poly(HPMA-co-MAGlyPheLeuGly-NHNH₂))

The polymerization procedure was the same as in Example 2a, the difference being again only in the composition of the polymerization mixture. The composition of the polymerization mixture was as follows:
HPMA 10 g (70 mmol), HPMA-*co*-MA-GlyPheLeuGly-NHNH₂ 2.5 g (5.6 mmol), azobis(isocyanovaleric acid) 1.125 g (1 wt %), and dimethylsulfoxide 100ml. The polymerization was carried out at 55 °C and completed after 18 hours. The polymer was isolated from the polymerization mixture by precipitation into a 20-fold excess of ethyl acetate. The polymer was purified by reprecipitation from methanol into ethyl acetate.
The yield was 9.75 g (78 %), the content of hydrazide groups 5.5 mol %, molecular weight M_{w} = 43200 g/mol, polydispersity index Iₙ = 2.1.

### Example 3: Preparation of polymeric conjugate PHPMA-AH-NH-N=DOX

Copolymers with DOX bound to a PHPMA carrier by a hydrolytically cleavable hydrazone bond were prepared by reaction of hydrazide-groups-containing copolymers poly(HPMA-coMA-AH-NHNH₂) with DOX.HCl in methanol, catalyzed by acetic acid.

A solution of 15.384 g of copolymer poly(HPMA-*co*-MA-AH-NHNH₂) in 92.1 ml of methanol (167 mg of polymer/ml) was placed into a thermostatted cell, in which 2.5 g of DOX.HCl (4.3 mmol) was placed. The inhomogeneous suspension was stirred in the dark at 25 °C, and, after one minute, 4.9 ml of acetic acid was added (total volume 116 ml). The suspension gradually dissolved in the course of the reaction, and after 22 hours of the reaction, the polymeric product was isolated from the homogeneous solution by precipitation into 11 of ethyl acetate; the precipitate of the polymeric drug was isolated by filtration in sintered glass filter S4, washed with 150 ml of ethyl acetate, and dried until a constant weight. The total amount of DOX was determined spectrally. *M̅_{w}* and *M̅ₙ* were determined by liquid chromatography (LC AKTA) with light scattering detection (DAWN DSP multi-angle detector, Wyatt).

Characterization of the polymeric drug. The total yield of the reaction binding the drug: 17.2 g (96 %), the total content of DOX: 11.3 wt %, free DOX: 1.52 % out of the total content of DOX.

### Example 4: The release of doxorubicin from polymeric conjugates

The release of doxorubicin from conjugates differing in the structure of the link (spacer) between the drug and the polymer was carried out by incubation in 0.1 M phosphate buffer containing 0.15 M NaCl at 37 °C. The pH of the buffer was adjusted to the conditions in cell endosomes, i.e. a slightly acid environment with pH 5.5. Aliquot parts of the incubation medium were sampled at the respective intervals, and the content of DOX was determined after adding a carbonate buffer (0.1 M Na₂CO₃ + 4 M NaCl), after extraction into chloroform, and after evaporating the solvent and transferring into a methanolic solution by means of HPLC (Shimadzu VP) using a reverse phase column (Tessek SGX C₁₈, 7 µm, 125 x 4 mm), eluent flow rate: 0.5 ml/min, gradient from 40 to 100 % of solution B in 35 minutes (solution A: 10 % methanol, 89.9 % water, 0.1 % trifluoroacetic acid (TFA); solution B: 89.9 % methanol; 10 % water; 0,1 % TFA). A fluorescence detector (Shimadzu RF-10AXL) (λ_{exc} = 480 nm, λₑₘ = 560 nm) was used for detection. The calibration curve was produced using doxorubicin.

The measurement results of the release of the drug from the conjugates differing in the structure of the link (spacer) used are shown in Fig. 1.

References:
T. Etrych, M. Jelínková, B. 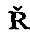 íhová, K. Ulbrich, New HPMA copolymers containing doxorubicin bound via pH sensitive linkage. Synthesis, *in vitro and in vivo* biological properties. J. Controlled Rel. 73, 89-102 (2001)
T. Etrych; P. Chytil, M. Jelínková, B. 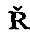 íhová, K. Ulbrich, Synthesis of HPMA CopolymersContaining Doxorubicin Bound via a Hydrazone Linkage. Effect of Spacer on Drug Releaseand in vitro Cytotoxicity. Macromolecular Biosci. 2, 43-52 (2002)
O. Hovorka, T. Etrych, M. 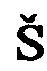 ubr, J. Strohalm, K. Ulbrich, B. 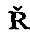 íhová, Differences in theIntracellular Fate of Free and Polymer-Bound Doxorubicin. J. Controlled Release 80, 101-117(2002)
J. Kope 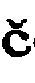 ek, P. Kope 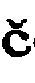 ková, T. Minko, Z. Lu, HPMA Copolymer-Anticancer Drug Conjugates:Design, Activity, and Mechanism of Action. Europ. J. Pharm. Biopharm. 50,61 - 81 (2000)
M. Ková 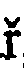 , L. Ková 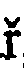 , V. 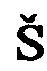 ubr, T. Etrych, K. Ulbrich, T. Mrkvan, J. Loucká, and B. 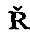 íhová,HPMA Copolymers Containing Doxorubicin Bound by Proteolytically or HydrolyticallyCleavable Bond: Comparison of Biological Properties In Vitro. J. Controlled Release 99, 301-314 (2004)
G. S. Kwon, Polymeric Drug Delivery Systems, Series: Drugs and the Pharmaceutical Sciences, Vol. 148, Dekker, Marcel Incorporated, 2005
H. Maeda, J. Wu, T. Sawa, Y. Matsumura, K. Hori, Tumor vascular permeability and the EPR effect in macromolecular therapeutics: a review. J Control Release 65, 271-284 (2000)
B. 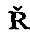 íhová, T. Etrych, M. Pechar, M. Jelínková, M. 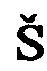 t'astný, O. Hovorka, M. Ková 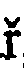 , K. Ulbrich, Doxorubicin Bound to a HPMA Copolymer Carrier Through Hydrazone Bond is Effective also in a Cancer Cell Line with a Limited Content of Lysosomes. J. Controlled Release 74,225-232 (2001)
K. Ulbrich, V. 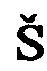 ubr, J. Strohalm, D. Plocová, M. Jelínková, B. 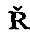 íhová, Polymeric Drugs Based on Conjugates of Synthetic and Natural Macromolecules I. Synthesis and Physicochemical Characterisation. J. Controlled Rel. 64, 63-79 (2000)
K. Ulbrich, T. Etrych, P. Chytil, M. Jelínková, B. 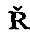 íhová, Antibody-Targeted Polymer-Doxorubicin Conjugates with pH-Controlled Activation. J. Drug Targeting, 12, 477-490(2004)
K. Ulbrich, T. Etrych, P. Chytil, M. Jelínková, B. 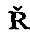 íhová, HPMA Copolymers with pH-Controlled Release of Doxorubicin. *In vitro* Cytotoxicity and *in vivo* Antitumor Activity. J. Controlled Release 87, 33-47 (2003)
K. Ulbrich, T. Etrych, P. Chytil, M. Pechar, M. Jelínková, B. 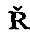 íhová, Polymeric Anticancer Drugs with pH-Controlled Activation. Int. J. Pharm. 277/1-2 67-72 (2004)
K. Ulbrich, T. Etrych, B. 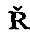 íhová, M. Jelínková, M. Ková 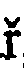 : pH-Sensitive polymeric conjugates of an anthracycline cancerostatic drug for targeted therapy. CZ 293787; WO 03/053473

## Claims

1. A method for the preparation of polymeric conjugates of N-(2-hydroxypropyl)methacrylamide and a methacryloylaminoacylhydrazone of doxorubicin with pH-controlled release of the drug, ***characterized by*** the following three steps of the synthesis:
a. preparation of a monomeric methacryloylaminoacylhydrazine, wherein the aminoacyl is derived from an amino acid or oligopeptide, by reaction of a methacryloyl halide with the respective peptide or amino acid and subsequent hydrazinolysis,
b. synthesis of a polymeric precursor by direct copolymerization of N-(2-hydroxypropyl)methacrylamide with the methacryloylaminoacylhydrazine, and
c. binding of doxorubicin to the polymeric precursor by reaction thereof with doxorubicin hydrochloride.

2. The method according to claim 1, ***characterized in* that** the acylation in step (1a) is carried out by reaction of the methyl ester hydrochloride of the respective amino acid or oligopeptide with methacryloyl chloride in a chlorinated hydrocarbon in the presence of anhydrous sodium carbonate.

3. The method according to claim 1 or 2, ***characterized in* that** the hydrazinolysis is carried out by reaction of the methyl ester of the methacryloylated amino acid or oligopeptide with hydrazine hydrate in the presence of a strong base.

4. The method according to any of the preceding claims, ***characterized in* that** in step (1b) radical copolymerization of N-(2-hydroxypropyl)methacrylamide with the methacryloylaminoacylhydrazine is carried out, initiated by thermally decomposable initiators based on azo or peroxy initiators, preferably azobis(isobutyronitrile), azobis(isocyanovaleric acid), or diisopropyl percarbonate.

5. The method according to claim 4, ***characterized in* that** the polymerization is carried out in a solvent selected from either lower C₁ to C₅ alcohols, or an aprotic polar solvent.

6. The method according to claim 5, ***characterized in* that** the solvent is selected from methanol, ethanol, dimethylformamide, or dimethylsulfoxide.

7. The method according to claim 6, ***characterized in* that** when the initiator is selected from azobis(isobutyronitrile) or azobis(isocyanovaleric acid), the polymerization is carried out at 45 to 70 °C, and when diisopropyl percarbonate is used as the initiator, the polymerization is carried out at 30 to 60 °C.

8. The method according to any of the preceding claims, ***characterized in* that** the reaction of the polymeric precursor in step (1c) is carried out in a solvent selected from anhydrous C₁ to C₅ alcohols or polar aprotic solvents, under catalysis by acetic acid, and the resulting conjugate is precipitated with ethyl acetate.

9. The method according to claim 8, ***characterized in* that** the solvent is selected from methanol, dried ethanol, dimethylformamide, or dimethylsulfoxide.

10. The method according to claim 9, ***characterized in* that** the starting concentration of the polymer is selected within the range of 100 to 190 mg/ml and the concentration of acetic acid within that of 30 to 80 g/ml.

11. The method according to claim 10, ***characterized in* that** the concentration of the polymer is 170 mg/ml and that of acetic acid 55 mg/ml at 25 °C.

12. The method according to any of the preceding claims, ***characterized in* that** the resulting product is purified by gel filtration.

## Patentansprüche

1. Verfahren zur Herstellung von polymeren Konjugaten des N-(2-Hydroxypropyl)methacrylamids und eines Methacryloylaminoacylhydrazons von Doxorubicin mit pH-gesteuerter Freisetzung des Arzneimittels, **gekennzeichnet durch** die folgenden drei Syntheseschritte:
(a) Herstellung eines monomeren Methacryloylaminoacylhydrazins, wobei sich das Aminoacyl von einer Aminosäure oder einem Oligopeptid ableitet, **durch** Reaktion eines Methacryloylhalogenids mit dem/der jeweiligen Peptid oder Aminosäure und anschliessende Hydrazinolyse,
(b) Synthese eines polymeren Precursors **durch** direkte Copolymerisation von N-(2-Hydroxypropyl)-methacrylamid mit einem Methacryloylaminoacylhydrazin und
(c) Binden von Doxorubicin an den polymeren Precursor **durch** Reaktion desselben mit Doxorubicin-Hydrochlorid.

2. Verfahren gemäss Anspruch 1, **dadurch gekennzeichnet, dass** die Acylierung im Schritt (1a) durch Reaktion des Methylester-Hydrochlorids der/des jeweiligen Aminosäure oder Oligopeptids mit Methacryloylchlorid in einem chlorierten Kohlenwasserstoff in Gegenwart von wasserfreiem Natriumcarbonat durchgeführt wird.

3. Verfahren gemäss Anspruch 1 oder 2, **dadurch gekennzeichnet, dass** die Hydrazinolyse durch Reaktion des Methylesters der/des methacryloylierten Aminosäure oder Oligopeptids mit Hydrazinhydrat in Gegenwart einer starken Base durchgeführt wird.

4. Verfahren gemäss einem der vorhergehenden Ansprüche, **dadurch gekennzeichnet, dass** im Schritt (1b) eine radikalische Copolymerisation von N-(2-Hydroxypropyl)methacrylamid mit einem Methacryloylaminoacylhydrazin durchgeführt wird, eingeleitet durch thermisch zersetzbare Initiatoren, basierend auf Azo- oder Peroxyinitiatoren, vorzugsweise Azobis(isobutyronitril), Azobis(isocyanovaleriansäure) oder Diisopropylpercarbonat.

5. Verfahren gemäss Anspruch 4, **dadurch gekennzeichnet, dass** die Polymerisation in einem Lösungsmittel, ausgewählt aus entweder niederen C₁₋₅-Alkoholen oder einem aprotischen polaren Lösungsmittel, durchgeführt wird.

6. Verfahren gemäss Anspruch 5, **dadurch gekennzeichnet, dass** das Lösungsmittel aus Methanol, Ethanol, Dimethylformamid oder Dimethylsulfoxid ausgewählt wird.

7. Verfahren gemäss Anspruch 6, **dadurch gekennzeichnet, dass**, wenn der Initiator aus Azobis(isobutyronitril) oder Azobis(isocyanovaleriansäure) ausgewählt ist, die Polymerisation bei 45 bis 70°C durchgeführt wird und, wenn Diisopropylpercarbonat als Initiator eingesetzt wird, die Polymerisation bei 30 bis 60°C durchgeführt wird.

8. Verfahren gemäss einem der vorhergehenden Ansprüche, **dadurch gekennzeichnet, dass** die Reaktion des polymeren Precursors im Schritt (1c) in einem Lösungsmittel, ausgewählt aus wasserfreien C₁₋₅-Alkoholen oder polaren aprotischen Lösungsmitteln, unter Essigsäure-Katalyse durchgeführt wird und das resultierende Konjugat mit Ethylacetat ausgefällt wird.

9. Verfahren gemäss Anspruch 8, **dadurch gekennzeichnet, dass** das Lösungsmittel aus Methanol, getrocknetem Ethanol, Dimethylformamid oder Dimethylsulfoxid ausgewählt wird.

10. Verfahren gemäss Anspruch 9, **dadurch gekennzeichnet, dass** die Ausgangskonzentration des Polymers innerhalb des Bereichs von 100 bis 190 mg/ml gewählt wird und die Konzentration der Essigsäure innerhalb des Bereichs von 30 bis 80 mg/ml liegt.

11. Verfahren gemäss Anspruch 10, **dadurch gekennzeichnet, dass** die Konzentration des Polymers 170 mg/ml ist und die Konzentration der Essigsäure bei 25°C 55 mg/ml ist.

12. Verfahren gemäss einem der vorhergehenden Ansprüche, **dadurch gekennzeichnet, dass** das resultierende Produkt durch Gelfiltration aufgereinigt wird.

## Revendications

1. Un procédé de préparation de conjugués polymères de N-(2-hydroxypropyl)méthacrylamide et de méthacxryloylaminoacylhydrazone de doxorubicine avec libération contrôlée du médicament en fonction du pH, **caractérisé par** les trois étapes suivantes de synthèse.
a. la préparation d'une méthacryloylaminoacylhydrazine monomérique, dans laquelle l'aminoacyle est dérivé d'un aminoacide ou d'un oligopeptide, par réaction d'un halogènure de méthacryloyle avec un peptide ou un aminoacide respectif et hydrazinolyse subséquente,
b. la synthèse d'un précurseur polymérique par copolymérisation directe de N-(2-hydroxypropyl)méthacrylamide avec la méthacryloylaminoacylhydrazine, et
c. la liaison de la doxorubicine au précurseur polymérique par réaction avec le chlorhydrate de doxorubicine.

2. Le procédé selon la revendication 1, **caractérisé en ce que** l'acylation dans l'étape (1a) est mise en oeuvre par réaction du chlorhydrate de l'ester méthylique de l'aminoacide ou de l'oligopeptide respectif avec le chlorure de méthacryloyle dans un hydrocarbure chloré en présence d'un carbonate de sodium anhydre.

3. Le procédé selon la revendication 1 ou 2, **caractérisé en ce que** l'hydrazinolyse est mise en oeuvre par réaction de l'ester méthylique de l'acide méthacryloyle ou de l'oligopeptide avec l'hydrate d'hydrazine en présence d'une base forte.

4. Le procédé selon l'une quelconque des revendications précédentes, **caractérisé en ce que**, est mise en oeuvre dans l'étape (1b) la copolymérisation radicalaire de la N-(2-hydroxypropyl)méthacrylamide avec la méthacryloylamino-acylhydrazine, laquelle polymérisation est initiée par des initiateurs thermiquement décomposables consistant en initiateurs azo ou péroxy, de préférence l'azobis(isobutyronitrile), l'acide azobis(isocyanovalérique) ou le percarbonate diisopropylique.

5. Le procédé selon la revendication 4, **caractérisé en ce que** la polymérisation est mise en oeuvre dans un solvant choisi parmi les alcools inférieurs en C₁ à C₅ ou un solvant aprotique polaire.

6. Le procédé selon la revendication 5, **caractérisé en ce que** le solvant est choisi parmi le méthanol, l'éthanol, le diméthylformamide ou le diméthylsulfoxyde.

7. Le procédé selon la revendication 6, **caractérisé en ce que**, lorsque l'initiateur consiste en azobis(isobutyronitrile) ou en l'acide azobis(isocyanovalérique), la polymérisation est mise en oeuvre entre 45 et 70°C, et **en ce que** lorsque du percarbonate de diisopropyl est utilisé en tant qu'initiateur, la polymérisation est mise en oeuvre entre 30 et 60°C.

8. Le procédé selon l'une quelconque des revendications précédentes, **caractérisé en ce que** la réaction du précurseur polymèrique dans l'étape (1c) est mise en oeuvre dans un solvant choisi parmi les alcools en C₁ à C₅ anhydres ou des solvants polaires aprotiques, sous catalyse en présence d'acide acétique, et le conjugat résultant est amené à précipiter dans l'acétate d'éthyle.

9. Le procédé selon la revendication 8, **caractérisé en ce que** le solvant est choisi parmi le méthanol, l'éthanol anhydre, le diméthylformamide ou le diméthylsulfoxyde.

10. Le procédé selon la revendication 9, **caractérisé en ce que** la concentration de départ du polymère est comprise entre 100 et 190 mg/ml et la concentration de l'acide acétique est comprise entre 30 et 80 mg/ml.

11. Le procédé selon la revendication 10, **caractérisé en ce que** la concentration en polymère est de 170 mg/ml et celle de l'acide acétique est de 55 mg/ml à 25°C.

12. Le procédé selon l'une quelconque des revendications précédentes, **caractérisé en ce que** le produit résultant est purifié par filtration sur gel.
